# FASCICULE DE BREVET EUROPEEN

(11) **EP 2 254 567 B1**
(45) Date de publication et mention de la délivrance du brevet: **30.04.2014**
(21) Numéro de dépôt: 09720199.0
(22) Date de dépôt: 25.02.2009
(51) Int. Cl.: A61K 31/185, A61P 25/00, A61P 27/02, A61K 31/315, A61K 9/08, A61K 9/20, A61K 9/48

(54) **N-ACETYL-TAURINATE DE ZINC POUR SON UTILISATION DANS UNE MÉTHODE DE PRÉVENTION ET/OU DE TRAITEMENT DES MALADIES AVEC ACCUMULATION DE LIPOFUSCINE**
ZINK-N-ACETYLTAURINAT ZUR VERWENDUNG BEI DER PRÄVENTION UND/ODER BEHANDLUNG VON ERKRANKUNGEN, DIE MIT LIPOFUSCIN AKKUMULATION VERBUNDEN SIND
ZINC N-ACETYLTAURINATE FOR USE IN THE PREVENTION AND/OR TREATMENT OF DISEASES ASSOCIATED WITH LIPOFUSCIN ACCUMULATION

(30) Priorité: 26.02.2008 FR 0851233
(43) Date de publication de la demande: 01.12.2010
(73) Titulaire: Tri-Inov, 92200 Neuilly sur Seine (FR)
(72) Inventeur: DURLACH, Jean, F-92200 Neuilly sur Seine (FR)
(74) Mandataire: Gillard, Marie-Louise
(86) Numéro de dépôt international: PCT/FR2009/050297
(87) Numéro de publication internationale: WO 2009/112758

(56) Documents cités:
- WO-A-2005/027950
- FR-A- 2 384 751
- US-A- 6 103 756
- US-A1- 2005 249 821
- CANGEMI FRANCIS E: "TOZAL Study: an open case control study of an oral antioxidant and omega-3 supplement for dry AMD." BMC OPHTHALMOLOGY 2007, vol. 7, no. 3, 2007, XP002496218 ISSN: 1471-2415
- HARRAKI B ET AL: "Effect of taurine, L-glutamine and L-histidine addition in an amino acid glucose solution on the cellular bioavailability of zinc." BIOMETALS : AN INTERNATIONAL JOURNAL ON THE ROLE OF METAL IONS IN BIOLOGY, BIOCHEMISTRY, AND MEDICINE JUL 1994, vol. 7, no. 3, juillet 1994 (1994-07), pages 237-243, XP002496219 ISSN: 0966-0844 cité dans la demande
- GOTTSCHALL-PASS K T ET AL: "Oscillatory potentials and light microscopic changes demonstrate an interaction between zinc and taurine in the developing rat retina." THE JOURNAL OF NUTRITION JUN 1997, vol. 127, no. 6, juin 1997 (1997-06), pages 1206-1213, XP002496220 ISSN: 0022-3166 cité dans la demande
- GRAHN BRUCE H ET AL: "Zinc and the eye" JOURNAL OF THE AMERICAN COLLEGE OF NUTRITION, AMERICAN COLLEGE OF NUTRION, WILMINGTON, NC, US, vol. 20, no. 2 with Supplement, 1 avril 2001 (2001-04-01), pages 106-118, XP002334806 ISSN: 0731-5724

## Description

La présente invention a pour objet une nouvelle utilisation du N-acétyl-taurinate de zinc.

Le N-acétyl-taurinate de zinc appartient à la famille des dérivés de la taurine à activité neuro-musculaire renforcée, qui sont décrits dans le brevet FR 2 384 751. On a maintenant trouvé de façon surprenante que le N-acétyl-taurinate de zinc (ATA-Zn) peut être utilisé pour prévenir et/ou traiter les maladies avec accumulation de lipofuscine.

L'accumulation de lipofuscine peut être due notamment au vieillissement ou à un stress oxydatif.

Le vieillissement est constitué par un ensemble de modifications fonctionnelles qui diminuent progressivement l'aptitude de la personne à assurer son équilibre physiologique. Une des manifestations du vieillissement est l'accumulation dans les tissus, de la lipofuscine, qui est une substance auto-fluorescente et non-dégradable ; elle constitue le stigmate majeur du vieillissement.

Le stress oxydatif (ou stress oxydant) est une agression des cellules et des tissus résultant d'un dérèglement des réactions d'oxydation dans les cellules qui entraîne la formation de substances toxiques, telles que les radicaux libres (ou espèces réactives de l'oxygène dénommées « Reactive Oxygen Species » (ROS)) dans les publications anglo-saxonnes). Le stress oxydatif peut toucher l'ensemble des tissus et des métabolismes, participer au veillisement et à un grand nombre de pathologies, telles que les maladies cardio-vasculaires (athéro-thrombose), les cancers, les maladies inflammatoires, les maladies métaboliques (diabète sucré, obésité) et les maladies dégénératives, telles que la maladie d'Alzheimer, la maladie de Parkinson, la cataracte ou la dégénérescence maculaire liée à l'âge. La dégénérescence maculaire liée à l'âge (DMLA) est la première cause de malvoyance dans les pays industrialisés chez les adultes ayant plus de 55 ans. Elle se manifeste par un épaississement de la zone maculaire de la rétine ou une atrophie de cette zone et parfois par des saignements. (Opthal. Physiol. Opt. 2003, 23 : 383-399 et Optometry in practice, vol.5 (2004) 15-32)

Le zinc est très concentré dans les tissus oculaires, en particulier dans la rétine et l'épithélium pigmentaire. Il agit comme cofacteur pour les enzymes antioxydantes (la déshydrogénase et la catalase rétinienne).

Les antioxydants, tels que les vitamines C et E, les caroténoïdes et le zinc peuvent limiter les risques de progression de la dégénérescence maculaire liée à l'âge.

En particulier, il a été démontré que le zinc joue un rôle important dans le maintien de la fonction oculaire normale et que l'utilisation du zinc comme complément alimentaire peut diminuer le processus de vieillissement chez les souris (Journal of the American college of nutrition, 2001, vol.20, n°2, 106-118). On sait également que le zinc semble avoir un rôle fondamental dans la prévention du diabète sucré et de ses complications (Li X et al. Diabetes and methallothionein. Mini-Reviews in Medical Chemistry 2007;7:761-768.) Cependant, l'utilisation du zinc en complément alimentaire doit être envisagée avec précaution car un excès de zinc peut entraîner des effets toxiques.

De plus, des études ont montré que la taurine interagit étroitement avec le zinc dans le développement de la rétine chez le rat (J. Nutr. 1997, Jun ; 127(6) :1206-13) et que la taurine en complément alimentaire, augmente l'absorption de zinc par les fibroblastes (Biometals, 1994, 7, 237-243).

On a maintenant trouvé de façon surprenante que le N-acétyl-taurinate de zinc peut prévenir et/ou traiter les maladies avec accumulation de lipofuscine due notamment au vieillissement ou à un stress oxydatif, en particulier les maladies de la rétine, notamment la dégénérescence maculaire liée à l'âge et la rétinopathie diabétique.

Ainsi, la présente invention a pour objet le N-acétyl-taurinate de zinc de formule :

[CH₃-CO-NH-CH₂-CH₂-SO₃]⁻₂Zn²⁺

pour son utilisation dans une méthode pour prévenir et/ou traiter les maladies avec accumulation de lipofuscine due notamment au vieillissement ou à un stress oxydatif, en particulier, les maladies de la rétine, notamment pour prévenir et/ou retarder la dégénérescence maculaire liée à l'âge et la rétinopathie diabétique.

L'invention a également pour objet l'utilisation du N-acétyl-taurinate de zinc de formule :

[CH₃-CO-NH-CH₂-CH₂-SO₃]⁻₂Zn²⁺

pour la préparation de médicaments utiles pour prévenir et/ou traiter les maladies liées à l'accumulation de lipofuscine due notamment au vieillissement ou à un stress oxydatif, en particulier, les maladies de la rétine, notamment pour prévenir et/ou retarder la dégénérescence maculaire liée à l'âge et la rétinopathie diabétique.

Le N-acétyl-taurinate de zinc est préparé par action de l'anhydride acétique sur la taurine en présence d'acétate de zinc selon un procédé analogue à celui décrit pour la préparation du N-acétyl-taurinate de sodium par M. TERAKOA (Hoppe-Seyler Zeitschrift für Physiologische Chemie, 145, 242 (1925)).

De préférence, on utilise le N-acétyl-taurinate de zinc dihydraté.

Pour l'administration aux patients souffrant de maladies liées à l'accumulation de lipofuscine due au vieillissement ou à un stress oxydatif, telles que notamment les maladies de la rétine, en particulier la dégénérescence maculaire liée à l'âge et la rétinopathie diabétique, le N-acétyl-taurinate de zinc est mélangé en tant que principe actif avec un excipient pharmaceutiquement acceptable couramment utilisé pour la préparation de compositions pharmaceutiques administrables par voie orale, parentérale ou locale.

Avantageusement, le N-acétyl-taurinate de zinc peut être présenté sous les formes administrables :
- par voie orale, telles que comprimés, dragées, capsules, gélules, sachets solutions, contenant le principe actif à la dose unitaire de 0,06 à 1 g et, pour les solutions de 0,6 à 5 g par 10 ml ;
- par voie parentérale, telles que solutions injectables conditionnées en ampoules, contenant de 0,1 à 2 g de principe actif par ampoule ;
- par voie locale, telles que lotions, crèmes, pommades, solutés, collyres, etc...

La dose à administrer journellement est généralement de 0,06 à 10 g et de préférence de 1 à 2 g.

L'activité de ATA-Zn sur le vieillissement a été mesurée par la détermination de la quantité de lipofuscine dans certains tissus chez les rats agés (rétine, prostate et hippocampe).

L'activité antioxydante du N-acétyl-taurinate de zinc a été démontrée en utilisant la lignée de cellules épithéliales pigmentées rétiniennes ARPE-19, qui constitue un outil couramment utilisé pour l'étude de la physiologie et de la pathologie de la rétine. Cette lignée cellulaire a été décrite par :
a) Dunn KC et al., "a human retinal pigment epithelial cell line with differentiated properties". Exp Eye Res. 1996;62:155-169;
b) Alizadeh M et al., "Downregulation of differentiation specific gene expression by oxidative stress in ARPE-19 cells". Invest Ophthalmol Vis Sci 2001; 42:2706-13
c) Hui Cai et al. "Gene expression profile of cultured adult compared to immortalized human retinal pigment epithelium" Molecular Vision 2006, 12: 1-14.
d) Ishida BY et al. High density lipoprotein mediated lipid efflux from retinal pigment epithelial cells in culture. Br J Ophtalmol 2006; 90:616-620.

Les tests réalisés reportés ci-après montrent que seul le N-acétyl-taurinate de zinc a un effet sur la production d'adénosine triphosphate (ATP) par les cellules ARPE-19 traitées par ATA-Zn, puis exposées à l'eau oxygénée (H₂O₂).

En revanche, le zinc seul ou la taurine seule n'ont aucun effet.

D'autres tests ont montré que l'effet du N-acétyl-taurinate de zinc sur la production d'ATP par les cellules ARPE-19 est quasi identique voire même supérieur à celui des antioxydants de référence, tels que l'acide docosahexaénoïque (DHA), la lutéine et la zéaxanthine. La lutéine et la zéaxanthine sont des pigments naturels de la famille des caroténoïdes qui sont concentrés dans la macula et la protègent des dommages causés par l'oxydation ou la lumière. La lutéine et la zéaxanthine sont des compléments alimentaires naturels utilisés dans le traitement de la dégénérescence maculaire liée à l'âge.

L'invention va être maintenant décrite plus en détail par la préparation et les tests ci-après.

### PREPARATION

On a mélangé 20,25 g de taurine et 17,5 g d'acétate de zinc pur sec et on a ajouté 50 g d'eau pure.

On a chauffé la suspension résultante à une température comprise entre 65 et 75°C et on a ajouté à cette suspension, 45 g d'anhydride acétique puis on a chauffé à 100-105°C. On a ensuite ajouté 100 ml d'éthanol anhydre au mélange réactionnel résultant à une température comprise entre 70 et 75°C.

On a finalement obtenu 30 ± 3 g du produit attendu sous la forme d'une poudre blanche soluble dans l'eau et peu soluble dans l'éthanol (rendement pondéral : 36,25 %).

### Analyse (en pourcentages)

| **Analyse** | **Calculé** | **Trouvé** |
|---|---|---|
| C | 24,16 | 22,75 |
| H | 4,05 | 4,60 |
| N | 7,04 | 5,73 |
| Zn* | 16,4 | 16,7 |

| | | |
|---|---|---|
| ** dosage de Zn par EDTA* | | |

### TESTS PHARMACOLOGIOUES

### I) Test in vivo

### a) Effet de /'ATA-Zn sur laccumulation intracellulaire de lipofuscine dans la rétine, la prostate et l'hippocampe de rats âgés.

Des rats albinos Sprague-Dawley males, âgés de 16 mois, ont été mis dans des cages en plastique standard, par groupe de 5, avec de l'eau et de la nourriture *ad libitum.* Les conditions de vie des animaux étaient les suivantes : Cycle de lumière jour:nuit, 12:12h, température constante de 22 ± 2°C et humidité relative de 60 %.

Pour le traitement chronique, on a administré aux rats, de l' ATA-Zn *per os*, une fois par jour pendant 4 semaines, à la dose de 40 mg/kg (n=6). Les animaux témoins ont reçu uniquement de l'eau (véhicule) pendant la même durée (n=6). 24 heures après la fin du traitement, les rats ont été anesthésiés avec de l'équithésine (2,5 mg/kg, i.p.) et perfusés par voie trans-cardiaque avec 1 % de paraformaldéhyde et 1,25 % de glutaraldéhyde dans un tampon cacodylate de sodium à 0,1 % (pH 7,4) obtenu à partir d'anhydride arsénieux et d'acétate de potassium.

Les échantillons des tissus prélevés (rétine, prostate et hippocampe) ont été ensuite coupés au cryostat en petits morceaux (1 mm³) et fixés dans un mélange de formaldéhyde et de glutaraldéhyde dans un tampon cacodylate pendant deux heures. Ensuite, les échantillons ont été fixés dans du tétraoxyde d'osmium deshydraté en concentration ascendante d'acétone et de toluène et enfin inclus dans des blocs de résine (résine EPON). Les morceaux de tissus ainsi traités ont été examinés au microscope à fluorescence BX-60 Olympus. La rétine, la prostate et l'hippocampe ont montré une accumulation claire et significative de lipofuscine.

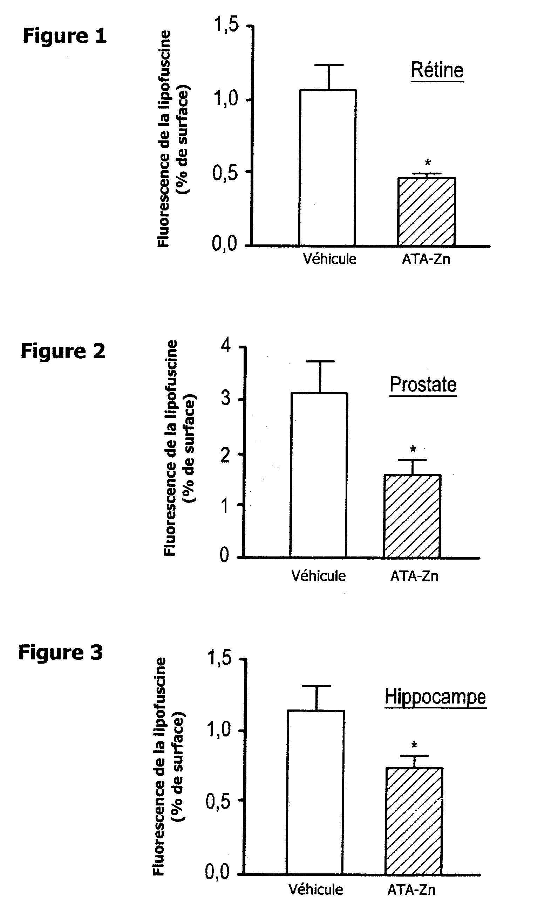

La fluorescence dans tous les tissus a été quantifiée en utilisant un système d'analyse d'image (KS 300 ; Karl Zeiss). L'analyse a été effectuée en mesurant le pourcentage de surface occultée par la présence de fluorescence et comparée à une zone standard de 2000 µm² (fluorescence de lipofuscine exprimée en % de surface par rapport à la zone standard) en utilisant un grossissement de l'objectif X40 et une caméra vidéo microscope digitale Zeiss Sound Vision (agrandissement total sur l'écran de l'ordinateur 7500X). Les valeurs mesurées dans les surfaces d'absence de fluorescence des morceaux de tissus, ont été soustraites comme bruit de fond de limage binaire résultante. L'analyse a été réalisée en prenant cinq morceaux différents par tissu (rétine, prostate et hippocampe) par animal et en choisissant 3 champs différents, au hasard, pour chaque morceau. Les résultats exprimés en moyenne ± écart standard et analysé par le test-t sont reportés sur les figures 1, 2 et 3 qui sont des histogrammes exprimant la fluorescence de la lipofuscine (% de surface) dans les tissus de la rétine (Figure 1) de la prostate (Figure 2) et de l'hippocampe (Figure 3) après administration du véhicule ou de l'ATA-Zn.

L'administration chronique de l'ATA-Zn à la dose de 40 mg/kg *per os* a produit une réduction significative du pourcentage de la surface occupée par la fluorescence (positive) de lipofuscine dans la rétine, la prostate et l'hippocampe, chez les rats âgés, par rapport aux animaux témoins.

Ce test montre que l' ATA-Zn peut inhiber l'accumulation de lipofuscine liée au vieillissement dans les tissus de la rétine, de l'hippocampe et de la prostate.

### II) Tests in vitro

### a) Matériels et méthodes

Dans les tests ci-après, on a utilisé la lignée de cellules épithéliales pigmentées rétiniennes humaines ARPE-19.

Pour évaluer le rôle protecteur de l'ATA-Zn, vis-à-vis d'un stress oxydant de l'épithélium rétinien, on a utilisé le modèle expérimental ci-après.

Des cellules ARPE-19 cultivées dans un milieu approprié [mélange milieu DMEM (Dulbecco's modified Eagle's medium) et milieu Ham's F12] contenant (10 % de sérum de veau foetal inactivé par la chaleur ; 2mM glutamine ; 0,1 mM de milieu essentiel minimal [référence ?]; une solution d'acides aminés non essentiels et du sulfate de gentamicine, dans des micro-plaques ayant 96 puits, ont été exposées à différentes concentrations d'eau oxygénée (H₂O₂) qui sont toxiques pour ces cellules. Les taux cellulaires d'ATP ont été mesurés en utilisant le test disponible dans le commerce qui mesure la production d'ATP « ATPLite-M » fabriqué par PERKIN ELMER à l'aide d'un lecteur multiplaques de PERKIN ELMER Life Sciences, Boston, MA. Ce test est basé sur le principe que la production d'ATP dépend de la viabilité des cellules et le fait que la diminution ou l'augmentation d'ATP reflète une altération correspondante de la viabilité des cellules.

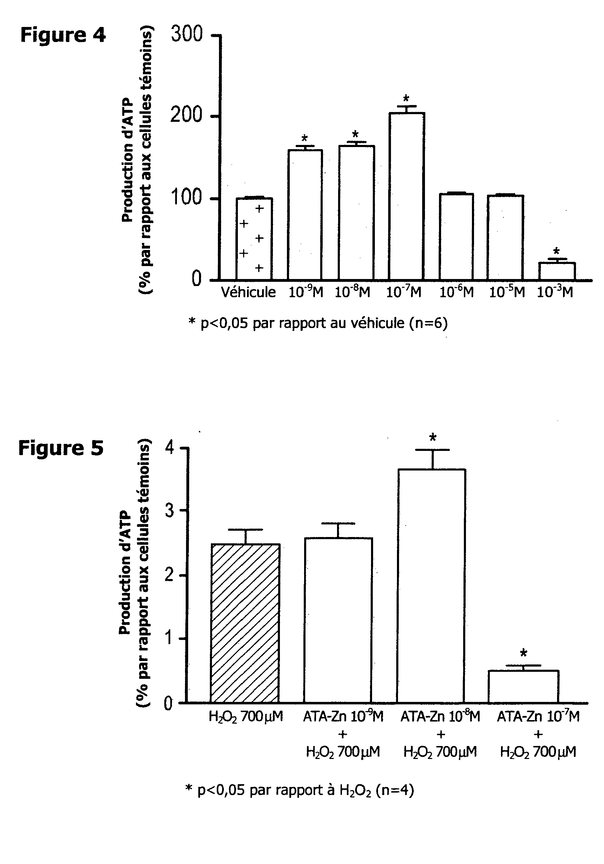

L'effet protecteur d'ATA-Zn a été testé en traitant les cellules ARPE-19 avec de l'ATA-Zn et en les exposant ultérieurement à l'eau oxygénée. Différents temps d'exposition et différentes doses ont été utilisées. Des tests comparatifs ont été également effectués. Les résultats de ces tests sont reportés sur les figures 5 à 9 qui sont des graphes donnant la production d'ATP, exprimées en pourcentage par rapport à la production de'ATP de cellules témoins (ayant reçu uniquement le véhicule) en fonction des concentrations d'H₂O₂, d'ATA-Zn ou des substances de comparaison.

### b) Test 1: détermination des doses efficaces de l'ATA-Zn et de sa toxicité.

Les cellules ARPE-19 ont été traitées avec différentes concentrations de l'ATA-Zn (10⁻³ à 10⁻⁹ M) dissous dans un véhicule constitué d'un milieu de culture exempt de sérum et contenant 0,01 % de DMS0 (concentration connue pour ne pas affecter la viabilité des cellules). La production d'ATP par les cellules ARPE-19 ainsi traitées a été déterminée 24 heures après l'addition de l'ATA-Zn. On a aussi déterminé la production d'ATP de cellules témoins traitées uniquement avec le milieu de culture exempt de sérum et contenant 0,01 % de DMSO (véhicule). Les résultats obtenus, reportés sur la figure 4, montrent que les concentrations faibles de ATA-Zn (10⁻⁹ M, 10⁻⁸M et 10⁻⁷M) sont capables d'induire une augmentation de la viabilité des cellules alors que des concentrations plus élevées (10⁻³M) sont toxiques pour ces cellules.

### c) Test 2 : effet cytoprotecteur anti-stress oxydatif

Dans ce test on a utilisé les concentrations de l'ATA-Zn qui induisent une production significative d'ATP par les cellules ARPE-19, puis, après 1,5 heure, on a soumis ces cellules à un traitement avec H₂O₂ à la dose toxique de 700 µM. Les résultats obtenus, rassemblés sur la figure 5, montrent que l'ATA-Zn est capable de protéger les cellules d'un stress oxydatif, seulement à la concentration de 10⁻⁸M alors qu'à la concentration de 10⁻⁹M, l'ATA-Zn n'est pas efficace, et qu'à la concentration de 10⁻⁷M, il potentialise même l'effet toxique de H₂O₂.

### d) Test 3 : effet cytoporotecteur de ATA-Zn sur les cellules soumises à un stress oxydatif.

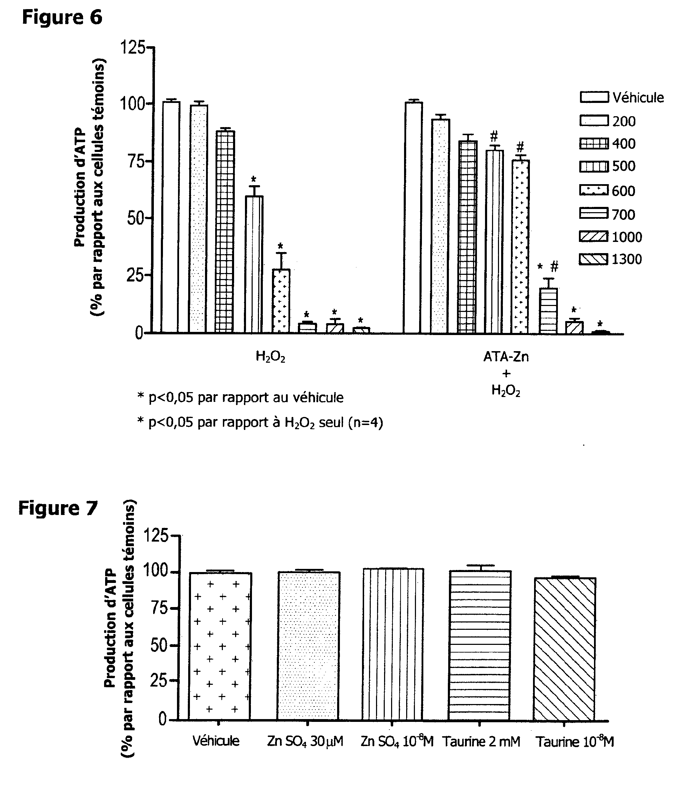

Les cellules ARPE-19 ont été exposées à différentes concentrations de H₂O₂ (exprimées en µM) en présence ou en l'absence de ATA-Zn à 10⁻⁸M (traitement une heure et demi avant l'exposition à H₂O₂). Les résultats reportés sur la figure 6 montrent que l'ATA-Zn est capable de protéger les cellules ARPE-19 d'un stress oxydatif induit par différentes concentrations de H₂O₂. En fait, les concentrations toxiques de H₂O₂ n'ont pas été capables d'affecter de manière significative la viabilité des cellules lorsque celles-ci ont été pré-traitées avec de l'ATA-Zn.

Les tests ci-dessus montrent que l'ATA-Zn peut exercer un rôle thérapeutique potentiel, dans le traitement des différentes pathologies de l'épithélium rétinien soumis à un stress oxydatif.

### e) Test comparatif n°1: détermination des doses efficaces de taurine et de Zn SO₄._

Dans ce test, on a comparé l'effet de la taurine simple ou N-acétylée et du sulfate de zinc sur la prolifération des cellules ARPE-19. La taurine a été utilisée à deux concentrations (2mM et 10⁻⁸M) et le sulfate de zinc a été utilisé aux concentrations ci-après : 30µm et 10⁻⁸M. Les résultats rassemblés sur la figure 7 montrent que ni le sulfate de zinc ni la taurine aux différentes concentrations utilisées n'ont été capables d induire un effet prolifératif significatif sur les cellules ARPE-19.

### f) Test comparatif n°2: détermination de l'effet cytoprotecteur en milieu oxydant.

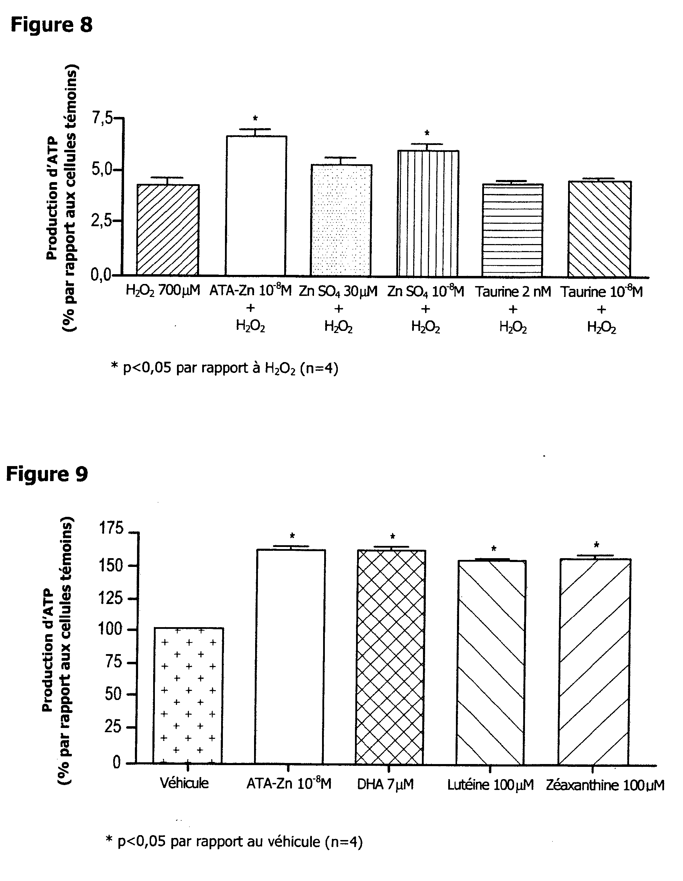

De manière à déterminer l'effet anti-oxydant du sulfate de zinc, de la taurine simple ou N-acétylée et de l' ATA-Zn, on a pré-traité les cellules ARPE-19 avec différentes concentrations de ZnSO₄, de taurine simple ou N-acétylée et de l' ATA-Zn. Ensuite, on a soumis les cellules pendant 24 heures à un traitement avec de l'eau oxygénée à 700 µM. Les résultats obtenus, qui sont rassemblés sur la figure 8, confirment la capacité de l'ATA-Zn à protéger les cellules ARPE-19 d'un stress oxydatif. On notera que le sulfate de zinc à la concentration de 10⁻⁸M induit un effet protecteur inférieur à celui de l' ATA-Zn. La taurine et le sulfate de zinc à la concentration de 30µM n'ont pas été capables de protéger les cellules contre le stress oxydant induit par H₂O₂.

### g) Test comparatif n°3: comparaison avec les anti-oxydants connus DHA lutéine et zéaxanthine.

Dans ce test on a comparé l'effet de l'ATA-Zn à celui obtenu avec le DHA, la lutéine et la zéaxanthine, qui sont couramment utilisés dans les études cliniques et pré-cliniques en particulier pour leurs propriétés antioxydantes et dans le traitement de la dégénérescence maculaire liée à l'âge. Les résultats de la figure 9 montrent que l'ATA-Zn a un effet comparable aux anti-oxydants connus.

## Revendications

1. Utilisation du N-acétyl-taurinate de zinc pour la préparation de médicaments utiles pour prévenir et/ou traiter les maladies avec accumulation de lipofuscine.

2. Utilisation selon la revendication 1 pour la préparation de médicaments utiles pour prévenir et/ou traiter les maladies avec accumulation de lipofuscine due au vieillissement.

3. Utilisation selon la revendication 1 pour la préparation de médicaments utiles pour prévenir et/ou traiter les maladies avec accumulation de lipofuscine due à un stress oxydatif.

4. Utilisation selon l'une quelconque des revendications 1 à 3, pour la préparation de médicaments utiles pour prévenir et/ou retarder la dégénérescence maculaire liée à l'âge et la rétinopathie diabétique.

5. Utilisation selon l'une quelconque des revendications 1 à 4 **caractérisée en ce que** le N-acétyl-taurinate est dihydraté.

6. Utilisation selon l'une quelconque des revendications 1 à 5, pour la préparation de médicaments pour l'administration orale.

7. Utilisation selon la revendication 6, pour la préparation de médicaments sous forme de gélules, sachets, comprimés ou solutions contenant 0,06 à 1 g par dose unitaire ou 0,6 à 5 g / 10 ml de solution.

8. Utilisation selon l'une quelconque des revendications 1 à 5, pour la préparation de médicaments pour l'administration parentérale.

9. Utilisation selon la revendication 8, pour la préparation de médicaments sous forme d'ampoules injectables contenant 0,1 à 2 g par ampoule.

10. Composition pharmaceutique pour utilisation dans une méthode de prévention et/ou de traitement des maladies avec accumulation de lipofuscine, **caractérisée en ce qu'**elle contient du N-acétyl-taurinate de zinc à titre de principe actif en combinaison avec un excipient pharmaceutiquement acceptable.

11. Composition pharmaceutique pour utilisation selon la revendication 10, **caractérisée en ce qu'**elle se présente sous la forme de gélules, sachets, comprimés ou solutions contenant 0,06 à 1 g par dose unitaire ou 0,6 à 5 g/10 ml de solution.

12. Composition pharmaceutique pour utilisation selon la revendication 10, **caractérisée en ce qu'**elle se présente sous la forme d'ampoules injectables contenant 0,1 à 2 g par ampoule.

13. N-acétyl-taurinate de zinc pour son utilisation dans une méthode de prévention et/ou de traitement des maladies avec accumulation de lipofuscine.

14. N-acétyl-taurinate de zinc pour son utilisation dans une méthode de prévention et/ou de traitement des maladies liées à l'accumulation de lipofuscine due au vieillissement.

15. N-acétyl-taurinate de zinc pour son utilisation dans une méthode de prévention et/ou de traitement des maladies liées à l'accumulation de lipofuscine due à un stress oxydatif.

16. N-acétyl-taurinate de zinc pour son utilisation dans une méthode pour prévenir et/ou retarder la dégénérescence maculaire liée à l'âge ou la rétinopathie diabétique.

## Patentansprüche

1. Verwendung von Zink-N-Acetyl-Taurinat für die Zubereitung von Arzneimitteln, die für die Prävention und/oder die Behandlung von Krankheiten mit Anhäufung von Lipofuszin von Nutzen sind.

2. Verwendung nach Anspruch 1, für die Zubereitung von Arzneimitteln, die für die Prävention und/oder die Behandlung von Krankheiten mit altersbedingter Anhäufung von Lipofuszin von Nutzen sind.

3. Verwendung nach Anspruch 1, für die Zubereitung von Arzneimitteln, die für die Prävention und/oder die Behandlung von Krankheiten mit durch einen oxidativen Stress bedingter Anhäufung von Lipofuszin von Nutzen sind.

4. Verwendung nach einem der Ansprüche 1 bis 3, für die Zubereitung von Arzneimitteln, die für die Prävention und/oder die Verzögerung der mit dem Alter verbundenen Makuladegeneration und der diabetischen Retinopathie von Nutzen sind.

5. Verwendung nach einem der Ansprüche 1 bis 4, **dadurch gekennzeichnet, dass** das N-Acetyl-Taurinat dihydriert ist.

6. Verwendung nach einem der Ansprüche 1 bis 5, für die Zubereitung von Arzneimitteln zur oralen Verabreichung.

7. Verwendung nach Anspruch 6, für die Zubereitung von Arzneimitteln in Form von Kapseln, Beuteln, Tabletten oder Lösungen, die 0,06 bis 1 g pro Einzeldosis oder 0,6 bis 5 g / 10 ml Lösung enthalten.

8. Verwendung nach einem der Ansprüche 1 bis 5, für die Zubereitung von Arzneimitteln zur parenteralen Verabreichung.

9. Verwendung nach Anspruch 8, für die Zubereitung von Arzneimitteln in Form von injizierbaren Ampullen, die 0,1 bis 2 g pro Ampulle enthalten.

10. Pharmazeutische Zusammensetzung zur Verwendung bei einem Verfahren zur Prävention und/oder zur Behandlung von Krankheiten mit Anhäufung von Lipofuszin, **dadurch gekennzeichnet, dass** sie Zink-N-Acetyl-Taurinat als Wirkstoff in Kombination mit einem pharmazeutisch akzeptablen Hilfsstoff enthält.

11. Pharmazeutische Zusammensetzung zur Verwendung nach Anspruch 10, **dadurch gekennzeichnet, dass** sie in Form von Kapseln, Beuteln, Tabletten oder Lösungen, die 0,06 bis 1 g pro Einzeldosis oder 0,6 bis 5 g / 10 ml Lösung enthalten, vorliegt.

12. Pharmazeutische Zusammensetzung zur Verwendung nach Anspruch 10, **dadurch gekennzeichnet, dass** sie in Form von injizierbaren Ampullen, die 0,1 bis 2 g pro Ampulle enthalten, vorliegt.

13. Zink-N-Acetyl-Taurinat für seine Verwendung bei einem Verfahren zur Prävention und/oder zur Behandlung von Krankheiten mit Anhäufung von Lipofuszin.

14. Zink-N-Acetyl-Taurinat für seine Verwendung bei einem Verfahren zur Prävention und/oder zur Behandlung von Krankheiten, die mit der altersbedingten Anhäufung von Lipofuszin verbunden sind.

15. Zink-N-Acetyl-Taurinat für seine Verwendung bei einem Verfahren zur Prävention und/oder zur Behandlung von Krankheiten, die mit der durch einen oxidativen Stress bedingten Anhäufung von Lipofuszin verbunden sind.

16. Zink-N-Acetyl-Taurinat für seine Verwendung bei einem Verfahren zur Prävention und/oder zur Verzögerung der mit dem Alter verbundenen Makuladegeneration oder der diabetischen Retinopathie.

## Claims

1. Use of zinc N-acetyltaurinate, for the manufacture of a medicament useful for the prevention and/or treatment of lipofuscin accumulation-related diseases.

2. Use as claimed in claim 1, for the manufacture of a medicament useful for the prevention and/or treatment of lipofuscin accumulation-related diseases resulting from aging.

3. Use as claimed in claim 1, for the manufacture of a medicament useful for the prevention and/or treatment of lipofuscin accumulation-related diseases resulting from oxidative stress.

4. Use as claimed in any one of claims 1 to 3, for the manufacture of a medicament useful for the prevention and/or the delaying of age-related macular degeneration and diabetic retinopathy.

5. Use as claimed in any one of claims 1 to 4, **characterized in that** said N-acetyltaurinate is N-acetyltaurinate dihydrate.

6. Use as claimed in any one of claims 1 to 5, for the manufacture of a medicament for oral administration.

7. Use as claimed in claim 6, for the manufacture of a medicament in the form of gel capsules, sachets, tablets or solutions containing 0.06 to 1 g per unit dose or 0.6 to 5 g/10 ml of solution.

8. Use as claimed in any one of claims 1 to 5, for the manufacture of a medicament for parenteral administration.

9. Use as claimed in claim 8, for the manufacture of a medicament in the form of injectable vials containing 0.1 to 2 g per vial.

10. A pharmaceutical composition for use in preventing and/or treating lipofuscin accumulation-related diseases, **characterized in that** it contains zinc N-acetyltaurinate as an active ingredient in combination with a pharmaceutically acceptable excipient.

11. The pharmaceutical composition for use as claimed in claim 10, **characterized in that** it is provided in the form of gel capsules, sachets, tablets or solutions containing 0.06 to 1 g per unit dose or 0.6 to 5 g/10 ml of solution.

12. The pharmaceutical composition for use as claimed in claim 10, **characterized in that** it is provided in the form of injectable vials containing 0.1 to 2 g per vial.

13. Zinc N-acetyltaurinate for its use in preventing and/or treating lipofuscin accumulation-related diseases.

14. Zinc N-acetyltaurinate for its use in preventing and/or treating lipofuscin accumulation-related diseases resulting from aging.

15. Zinc N-acetyltaurinate for its use in preventing and/or treating lipofuscin accumulation-related diseases resulting from oxidative stress.

16. Zinc N-acetyltaurinate for its use in preventing and/or delaying age-related macular degeneration or diabetic retinopathy.
